# EUROPEAN PATENT APPLICATION

(11) **EP 2 281 501 A2**
(43) Date of publication of application: **09.02.2011**
(21) Application number: 10171055.6
(22) Date of filing: 28.07.2010
(51) Int. Cl.: A61B 3/113, G02C 13/00

(54) **Downward eyeball movement measuring jig, jig affixing device, and downward eyeball movement measuring method**

(30) Priority: 30.07.2009 JP 2009177443; 24.11.2009 JP 2009266442
(71) Applicant: Seiko Epson Corporation, Shinjuku-ku Tokyo (JP)
(72) Inventor: Wada, Osamu, Nagano 392-8502 (JP)
(74) Representative: HOFFMANN EITLE

(57) **Abstract**

A downward eyeball movement measuring jig which measures downward eyeball movement corresponding to a distance between a distant eye point and a near eye point of a spectacle lens attached to a spectacle frame to be worn by a wearer of the spectacle lens includes: a mask which is provided on the surface of an examination spectacle lens attached to the spectacle frame and has a reference line passing the distant eye point and extending in the horizontal direction.

## Description

### BACKGROUND

### 1. Technical Field

The present invention relates to a downward eyeball movement measuring jig, a jig affixing device, and a downward eyeball movement measuring method for a spectacle lens.

### 2. Related Art

When viewing an object, a person rotates his or her head or eyes to see the object from the side or the upper or lower part (viewing action). The rotation angle of the head or the eyes in the viewing action is peculiar to each person. Thus, various designing methods and adjusting methods for a spectacle lens considering the viewing action of each person have been proposed (for example, see JP-T-2003-523244 and JP-T-2008-521027).

A progressive-power lens is an aspherical lens which includes a distance portion having a power (dioptric power) corresponding to long-distance view and a near portion having a power corresponding to short-distance view. The distant portion is located at an upper position of the lens, and the near portion is located at a lower position of the lens. The progressive-power lens further includes a progressive band disposed between the distant portion and the near portion as a band having progressively varying power. These areas have no boundaries between one another, and both a distant object and a near object can be seen through one lens. The distant portion, the near portion, and the progressive band need to be adjusted according to the use purpose of each person (such as long-short distance view weighted, middle-short distance view weighted, short-short distance view weighted, full-time use, part-time use, static use, and dynamic use). This adjustment is called optical fitting. In case of the progressive-power lens, downward eyeball movement is an important factor for the optical fitting. The downward eyeball movement is a distance between a distant eye point as a position of vision on the lens in the condition of horizontal view by a wearer wearing the spectacle lens and a near eye point as a position of vision on the lens in the condition of short distance view by the wearer.

For adjustment of spectacles for the wearer, engineering fitting for adjusting spectacle information (eye examination information, spectacle frame information, and spectacle lens information) to respective parts of the wearer (such as shape and size of the face, thickness of the neck, relative positions and shapes of the nose, ears, and eyes) is performed in case of a single-vision lens. As for the progressive-power lens, however, the optical fitting described above needs to be conducted as well as the engineering fitting.

According to the technologies disclosed in JP-T-2003-523244 and JP-T-2008-521027, a head and eyeball movement measuring device is used to obtain quantitative values, and an optimal lens for each person is produced based on the obtained values. However, since the position of the wearer is also an important factor for determining the near eye point of the progressive-power lens as well as the head and eyeball movements, it is difficult to determine a highly accurate position of the near eye point in some cases.

Moreover, measurement of the eyeball movement disclosed in JP-T-2003-523244 and JP-T-2008-521027 imposes too heavy burden of examination time on a customer, and also requires a too expensive examination device.

### SUMMARY

It is an advantage of some aspects of the invention to provide a downward eyeball movement measuring jig, a jig affixing device, and a downward eyeball movement measuring method capable of measuring downward eyeball movement associated with a progressive-power lens with ease and high accuracy and at low cost.

A first aspect of the invention is directed to a downward eyeball movement measuring jig which measures downward eyeball movement corresponding to a distance between a distant eye point and a near eye point of a spectacle lens attached to a spectacle frame to be worn by a wearer of the spectacle lens including a mask which is provided on the surface of an examination spectacle lens attached to the spectacle frame and has a reference line passing the distant eye point and extending in the horizontal direction.

For measuring the downward eyeball movement, the examination spectacle lens is attached to the spectacle frame to be purchased by a consumer at a shop or the like, and the examination spectacle lens is measured by using the downward eyeball movement measuring jig according to the first aspect of the invention. The examination spectacle lens is made of acrylic resin having no power, for example.

According to this aspect of the invention, the downward eyeball movement measuring jig has the mask affixed to the examination spectacle lens to measure the downward eyeball movement for each person. The mask has the reference line, and the measurement is performed by disposing the mask such that the reference line agrees with the distant eye point of the spectacle lens. The horizontal direction herein refers to the horizontal direction for the wearer wearing the spectacle lens to which the mask is attached.

In this structure, the distant eye point is fixed. Thus, the downward eyeball movement can be measured by determining the position of the vision of the wearer in the condition of short-distance view.

Accordingly, the examination spectacle lens is attached to the spectacle frame to be worn by the wearer, and the examination spectacle lens is measured by using the mask. Thus, highly accurate measurement can be performed considering not only the viewing action of the wearer (movements of the eyes and the head) but also the position and behavior of the wearer seeing an object.

In addition, it is only required to determine the position receiving the vision of the wearer on the examination spectacle lens. Thus, the measurement can be easily performed with no heavy burden imposed on the wearer and at low cost with no necessity for using high-cost equipment.

According to the downward eyeball movement measuring jig of the first aspect of the invention, it is preferable that the mask has a width corresponding to the expected length of the downward eyeball movement of the wearer and two opposed sides. In this case, the reference line corresponds to one of the two opposed sides of the mask.

According to this structure, the mask has the two opposed sides, and one of the two sides is aligned with the position passing the distant eye point. In this arrangement, the optimum mask for the wearer can be selected by determining whether the other side agrees with the near eye point. It is considered that the other side agrees with the near eye point when the vision of the wearer in the condition of short-distance view (downward view) is not blocked by the mask on the examination spectacle lens. However, the agreement is not admitted when the distance between the other side and the near eye point is too long. Based on this determination, the mask having the optimum width for the wearer can be selected for measurement of the downward eyeball movement as the length corresponding to the width of the selected mask.

Accordingly, the examination spectacle lens is attached to the spectacle frame to be worn by the wearer, and the examination spectacle lens is measured by using the mask. Thus, highly accurate measurement can be performed considering not only the viewing action of the wearer (movements of the eyes and the head) but also the position and behavior of the wearer seeing an object.

In addition, it is only required to determine the position of the vision of the wearer on the examination spectacle lens. Thus, the measurement can be easily performed with no heavy burden imposed on the wearer and at low cost with no necessity for using high-cost equipment.

The downward eyeball measuring jig according to this aspect of the invention may be used for measuring the spectacle lens designed based on the measured downward eyeball movement. In this case, the downward eyeball measuring jig is employed to examine (check) whether the lens design is suited for the wearer.

According to the downward eyeball movement measuring jig of the first aspect of the invention, it is preferable that the mask has two opposed sides. In this case, the reference line is disposed at a position away from the two opposed sides.

According to this structure, the mask has the two opposed sides, and the reference line is located away from the two sides. Thus, the reference line is not any of the two sides forming the mask but a straight line provided on the mask.

In this case, the mask is disposed such that the reference line agrees with the distant eye point of the examination spectacle lens, and the examination spectacle lens is attached to the spectacle frame to be worn by the wearer. Then, the measurement is conducted by using the mask provided on the examination spectacle lens. Thus, highly accurate measurement can be performed considering not only the viewing action of the wearer (movements of the eyes and the head) but also the position and behavior of the wearer seeing an object.

According to the downward eyeball movement measuring jig of the first aspect of the invention, it is preferable that the other side of the two opposed sides of the mask is disposed close to the near eye point. In this case, the mask is a tape-shaped mask which has a tape base, and an adhesive portion disposed on the side of the tape base facing the examination spectacle lens and detachably affixed to the examination spectacle lens.

According to this structure, the tape-shaped mask can be detachably attached to the examination spectacle lens by the function of the adhesive portion. Thus, affixing and removing the tape-shaped mask can be facilitated.

Accordingly, a shop assistant at a shop or the like can easily conduct measurement and examination without using particular techniques. Moreover, cost reduction can be achieved by using the low-cost tape-shaped mask.

According to the downward eyeball movement measuring jig of the first aspect of the invention, it is preferable that the mask has a plurality of lines provided on the surface of the examination spectacle lens in an area expected to contain the near eye point in such directions parallel with the reference line. In this case, the mask is a tape-shaped mask which has a tape base, and an adhesive portion disposed on the side of the tape base facing the examination spectacle lens and detachably affixed to the examination spectacle lens.

According to this structure, the plural lines are provided in the area expected to contain the near eye point. Thus, it is only required for the wearer to determine which line agrees with or lies closest to the vision in the actual condition of short-distance view (near eye point) for the measurement. The distance between the selected line and the reference line corresponds to the downward eyeball movement.

Accordingly, it is only required for the wearer to determine which of the plural lines provided on the mask agrees with the vision in the condition of short-distance view (near eye point). Thus, the measurement can be easily performed with no heavy burden imposed on the wearer.

Moreover, plural selections are provided for the near eye point. Thus, the position of the near eye point can be more precisely determined, and the accuracy of the measurement can be raised.

According to the downward eyeball movement measuring jig of the first aspect of the invention, it is preferable that the plural lines have colors different from one another.

According to this structure, the plural lines have colors different from one another. Thus, the wearer can easily recognize the colors, and thus can easily determine which line agrees with or lies closest to the near eye point. It is preferable that the plural lines have opaque different colors and are disposed at equal intervals.

A second aspect of the invention is directed to a jig affixing device which affixes the downward eyeball movement measuring jig described above to an examination spectacle lens including: a spectacle hold unit which holds spectacles having the examination spectacle lens attached to a spectacle frame; and a tape supply unit which supplies the tape-shaped mask to the spectacles held by the spectacle hold unit. The spectacle hold unit has a base opposed to the examination spectacle lens, and a frame support member attached to the base in such a condition as to be movable in a direction perpendicular to a horizontal vision area passing an aye point of the examination spectacle lens and extending in the horizontal direction to support the spectacle frame. The tape supply unit has a cutter member which cuts the tape-shaped mask, and a lens affixing member which transfers the tape-shaped mask cut by the cutter member to the spectacles held by the spectacle hold unit and affixes the tape-shaped mask such that one of the sides of the tape-shaped mask can be aligned with the horizontal vision area.

According to this structure, the spectacles having the examination spectacle lens attached to the spectacle frame is held by the spectacle hold unit, the tape-shaped mask is formed by the tape supply unit, and the formed tape-shaped mask is affixed to the examination spectacle lens of the spectacles held by the spectacle hold unit.

The position of the held spectacles is adjusted by moving the frame support member provided on the spectacle hold unit. Thus, the distant eye point of the examination spectacle lens can be easily aligned with the one side of the tape-shaped mask.

The tape supply unit has the lens affixing member which transfers the tape-shaped mask cut by the cutter member and affixes the tape-shaped mask to the examination spectacle lens. Thus, the tape-shaped mask can be affixed to the optimum position on the examination spectacle lens only by setting a tape available on the market, for example. The horizontal vision area passing the eye point of the examination spectacle lens and extending in the horizontal direction herein refers to an area visible for the wearer in the left-right direction on a line passing the distant eye point and extending in the left-right direction of the spectacles.

According to this structure, the measuring jig constituted by the tape-shaped mask can be easily affixed to the examination spectacle lens. Thus, the measurement and examination can be easily conducted at a shop or the like.

According to the downward eyeball movement measuring jig of the first aspect of the invention, it is preferable that an end of the tape-shaped mask is formed by folding the adhesive portion such that the folded parts of the adhesive portion can be stacked with each other.

According to this structure, the end of the tape-shaped mask is folded. Thus, a folded area having no exposure of the adhesive portion can be formed. This area having no exposure of the adhesive portion can be held when the tape-shaped mask is affixed to or removed from the examination spectacle lens. Thus, the affixing and removing operations can be facilitated.

According to the jig affixing device of the second aspect of the invention, it is preferable that the cutter member has a fold forming portion which folds the end of the tape-shaped mask.

According to this structure, a folded area having no exposure of the adhesive portion can be formed at the end of the tape-shapedmask by using the fold forming portion. Thus, the subsequent transferring and affixing operations can be facilitated.

Particularly, the step for forming the tape-shaped mask can be shortened when the folded area is formed by the fold forming portion simultaneously with the cutting of the tape.

A third aspect of the invention is directed to a jig affixing device including: a spectacle hold stand which holds a measurement tape having a pair of the tape-shaped masks and the spectacles; a lens carrying portion made of low impact resilience material and disposed on the upper surface of the spectacle hold stand at a position corresponding to the examination spectacle lens; and a frame support member attached to the upper surface of the spectacle hold stand in such a condition as to be movable in a direction perpendicular to the horizontal direction of the examination spectacle lens to support the spectacle frame.

According to this structure, the lens carrying portion made of low impact resilience material is provided on the upper surface of the spectacle hold stand. The lens carrying portion is constituted by a pair of low impact resilience members corresponding to the two examination spectacle lenses.

Thus, the tape-shaped masks contained in the measurement tape can be affixed to the examination spectacle lenses by placing the measurement tape and the spectacles on the lens carrying portion and pressing the spectacles against the lens carrying portion.

In this case, the Measuring jig constituted by a pair of the tape-shaped masks can be easily affixed to the examination spectacle lenses. Thus, the measurement can be easily conducted at a shop or the like.

According to the jig affixing device of the third aspect of the invention, it is preferable that the measurement tape has a base film and a cover film. In this case, the measurement tape has a pair of the tape-shaped masks disposed between the base film and the cover film.

According to this structure, the measurement tape has the tape-shaped masks between the base film and the cover film. Since the measurement is executed for each of the two examination spectacle lenses, a pair of the tape-shaped masks are needed. The pair of the tape-shaped masks are fixed to the individual positions between the base film and the cover film. Thus, the tape-shaped masks need not be separately positioned when affixed to the examination spectacle lenses. Accordingly, the tape-shaped masks can be easily affixed to the spectacle lenses.

Moreover, the tape-shaped masks are covered by the cover film. Thus, the adhesives applied to the surfaces of the tape-shaped masks are not exposed. Accordingly, the tape-shaped masks can be easily handled, and thus the distribution of the tape-shaped masks can improve.

A fourth aspect of the invention is directed to a method for measuring downward eyeball movement corresponding to a distance between a distant eye point and a near eye point of a spectacle lens attached to a spectacle frame to be worn by a wearer of the spectacle lens including: preparing a tape-shaped mask which has a width corresponding to the expected length of the downward eyeball movement of the wearer for the surface of an examination spectacle lens attached to the spectacle frame, positioning the tape-shaped mask such that one of two opposed sides of the tape-shaped mask passes the distant eye point of the examination spectacle lens, and affixing the tape-shaped mask to the examination spectacle lens with the other side of the mask-shaped shape located close to the near eye point of the examination spectacle lens; and determining whether the near eye point of the examination spectacle lens agrees with the other side of the tape-shaped mask, which determination is made by the wearer wearing spectacles which has the examination spectacle lens attached to the spectacle frame with the tape-shaped mask affixed to the examination spectacle lens. When it is determined that the near eye point does not agree with the other side of the tape-shaped mask, determination is repeated after removing the tape-shapedmask from the examination lens and affixing another tape-shaped mask having a different width.

According to this method, the tape-shaped mask having the width corresponding to the expected length of the downward eyeball movement of the wearer is affixed to the examination spectacle lens. In this step, the tape-shaped mask is affixed to the examination spectacle lens under the condition that the one side of the tape-shaped mask agrees with the horizontal vision area passing the distant eye point and extending in the horizontal direction with the other side located close to the near eye point.

Then, the wearer wears the spectacles to which the tape-shaped mask has been affixed, and determines whether the vision in the condition of short-distance view (near eye point) agrees with the other side of the tape-shaped mask. This determination may be made by the wearer or by a person practicing the measurement. The agreement between the near eye point and the other side of the tape-shaped mask is admitted when the vision passes the examination spectacle lens.

When it is determined that the vision in the condition of short-distance view (near eye point) is blocked by the tape-shaped mask and does not pass the examination spectacle lens, the determining step is repeated after removing the tape-shaped mask from the examination spectacle lens and affixing another tape-shaped mask having a smaller width in the similar manner.

When it is determined that the near eye point agrees with the other side of the tape-shaped mask, the width of this tape-shaped mask corresponds to the length of the downward eyeball movement.

According to this method, the examination spectacle lens is attached to the spectacle frame to be worn by the wearer, and the examination spectacle lens is measured by using the mask. Thus, highly accurate measurement can be performed considering not only the viewing action of the wearer (movements of the eyes and the head) but also the position and behavior of the wearer seeing an object.

Moreover, it is only required to determine whether the vision of the wearer passes through the examination spectacle lens for the measurement. Thus, the measurement can be easily performed with no heavy burden imposed on the wearer and at low cost with no necessity for using high-cost equipment.

In addition, the tape-shaped mask used as the measuring jig can be easily affixed to or removed from the examination spectacle lens.

Accordingly, a shop assistant at a shop or the like can easily conduct measurement and examination without using particular techniques. Also, cost reduction can be achieved by using the low-cost tape-shaped mask.

A fifth aspect of the invention is directed to a method for measuring downward eyeball movement corresponding to a distance between a distant eye point and a near eye point of a spectacle lens attached to a spectacle frame to be worn by a wearer of the spectacle lens including: positioning the spectacle frame such that the reference line provided on the tape-shaped mask agrees with the distant eye point by using the jig affixing device of the third aspect of the invention, and affixing the tape-shaped mask to the examination spectacle lens by pressing the examination spectacle lens against the measurement tape; and determining which line of the plural lines provided on the tape-shaped mask agrees with the near eye point of the examination spectacle lens, the determination is made by the wearer wearing spectacles which has the examination spectacle lens attached to the spectacle frame with the tape-shaped mask affixed to the examination spectacle lens.

According to this method, the spectacle frame is positioned such that the reference line provided on the tape-shaped mask agrees with the distant eye point of the examination spectacle lens, and the tape-shaped mask is affixed to the examination spectacle lens by pressing the examination spectacle lens against the measurement tape.

Then, the wearer wears the spectacles to which the tape-shaped mask has been affixed and determines which line of the plural lines provided on the tape-shaped mask agrees with or lies closest to the vision in the condition of short-distance view (near eye point).

According to this method, the examination spectacle lens is attached to the spectacle frame to be worn by the wearer, and the examination spectacle lens is measured by using the tape-shaped mask. Thus, highly accurate measurement can be performed considering not only the viewing action of the wearer (movements of the eyes and the head) but also the position and behavior of the wearer seeing an object.

Moreover, it is only required to determine which line agrees with or lies closest to the vision for the measurement. Thus, the measurement can be easily performed with no heavy burden imposed on the wearer and at low cost with no necessity for using high-cost equipment.

### BRIEF DESCRIPTION OF THE DRAWINGS

The invention will be described with reference to the accompanying drawings, wherein like numbers reference like elements.

Fig. 1 schematically illustrates a progressive-power lens to which a downward eyeball movement measuring jig according to a first embodiment of the invention is affixed.

Fig. 2 is a plan view schematically illustrating a part of the structure of a jig affixing device according to the first embodiment.

Fig. 3 is a front view schematically illustrating the structure of the jig affixing device according to the first embodiment.

Figs, 4A through 4D illustrate steps for cutting a tape-shaped mask according to the first embodiment.

Figs. 5A through 5C illustrate steps for affixing the downward eyeball movement measuring jig according to the first embodiment.

Figs. 6A and 6B illustrate measurement of downward eyeball movement with the tape-shaped masks affixed according to the first embodiment.

Figs. 7A and 7B illustrate measurement of downward eyeball movement with the tape-shaped masks affixed according to the first embodiment.

Fig. 8 is a plan view schematically illustrating downward eyeball movement measuring jigs affixed to examination spectacle lenses according to a second embodiment of the invention.

Fig. 9 is a plan view schematically illustrating the structure of a measurement tape containing the downward eyeball movement measuring jigs according to the second embodiment.

Fig. 10 is a cross-sectional view taken along a line X-X in Fig. 9.

Figs. 11A and 11B schematically illustrate the structure of a jig affixing device according to the second embodiment, wherein: Fig. 11A is a plan view; and Fig. 11B is a cross-sectional view taken along a line XI-XI in Fig. 11A.

Figs. 12A through 12C illustrate steps for affixing the downward eyeball movement measuring jigs to the examination spectacle lenses according to the second embodiment.

Fig. 13 is a cross-sectional view illustrating the downward eyeball movement measuring jigs affixed to the examination spectacle lenses according to the second embodiment.

Fig. 14 illustrates lines actually viewed by a wearer according to the second embodiment.

Fig. 15 illustrates lines actually viewed by the wearer according to the second embodiment.

Figs. 16A and 16B schematically illustrate a jig affixing device according to a modified example of the second embodiment, wherein Fig. 16A is a plan view; and Fig. 16B is a cross-sectional view taken along a line XVI-XVI in Fig. 16A.

Fig. 17 is a plan view schematically illustrating downward eyeball movement measuring jigs according to a modified example of the second embodiment.

Fig. 18 is a plan view schematically illustrating a measurement tape containing downward eyeball movement measuring jigs according to a modified example of the second embodiment.

### DESCRIPTION OF EXEMPLARY EMBODIMENTS

### First Embodiment

A first embodiment of the invention is hereinafter described with reference to the drawings.

For measuring downward eyeball movement, an examination spectacle lens is attached to a spectacle frame to be purchased by a consumer at a shop or the like, and downward eyeball movement associated with the examination spectacle lens is measured by using a downward eyeball movement measuring jig. The examination spectacle lens is made of acrylic resin or the like having no power. After measurement, a spectacle lens is designed based on the measured downward eyeball movement. The spectacle lens thus designed is a progressive-power lens. For checking whether the progress-power lens has been designed in accordance with the measurement result, the downward eyeball movement measuring jig according to this embodiment can be attached to the designed spectacle lens.

In this embodiment, the vertical direction for the wearer wearing the spectacles corresponds to the up-down direction, and the horizontal direction for the wearer wearing the spectacles corresponds to the left-right direction.

### 1. Spectacle Lens

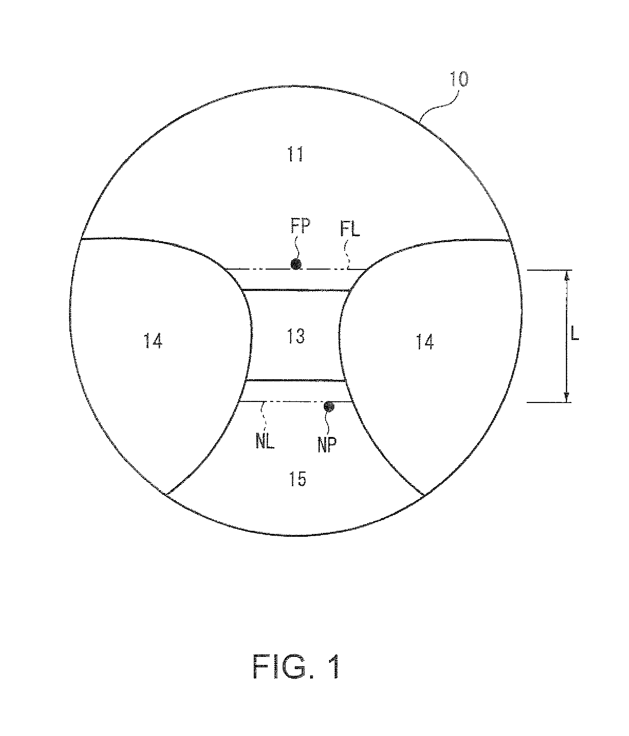

As illustrated in Fig. 1, a progressive-power lens as a spectacle lens 10 includes a distant portion 11 located at an upper position, a near portion 15 located at a lower position, a progressive band 13 interposed between the distant portion 11 and the near portion 15, and side portions 14 disposed adjacent to the sides of the progressive band 13.

The distant portion 11 has average dioptric power as relatively low plus power to be suited for long distance view. Particularly, the distant portion 11 has a distant eye point FP which receives a horizontal line passing the pupil center (i.e. , the vision) when the wearer of the spectacle lens 10 sees the front, and a distant eye point line FL as a line segment passing the distant eye point FP and extending in the left-right direction in the distant portion 11.

The near portion 15 has average dioptric power as relatively high plus power to be suited for short-distance view (such as for reading). Particularly, the near portion 15 has a near eye point NP which receives the vision when the wearer sees the near area (lower area), and a near eye point line NL as a line segment passing the near eye point NP and extending in the left-right direction in the near portion 15.

In this embodiment, a distance (length) L between the distant eye point line FL and the near eye point line NL in the up-down direction is measured as the downward eyeball movement.

The progressive-power band 13 is an area where the relative plus addition power progressively varies between the distant portion 11 and the near portion 15.

The side portions 14 are areas called astigmatism portions. An object is viewed double through the side portions 14. Thus, the wearer generally does not use the side portions 14 for viewing.

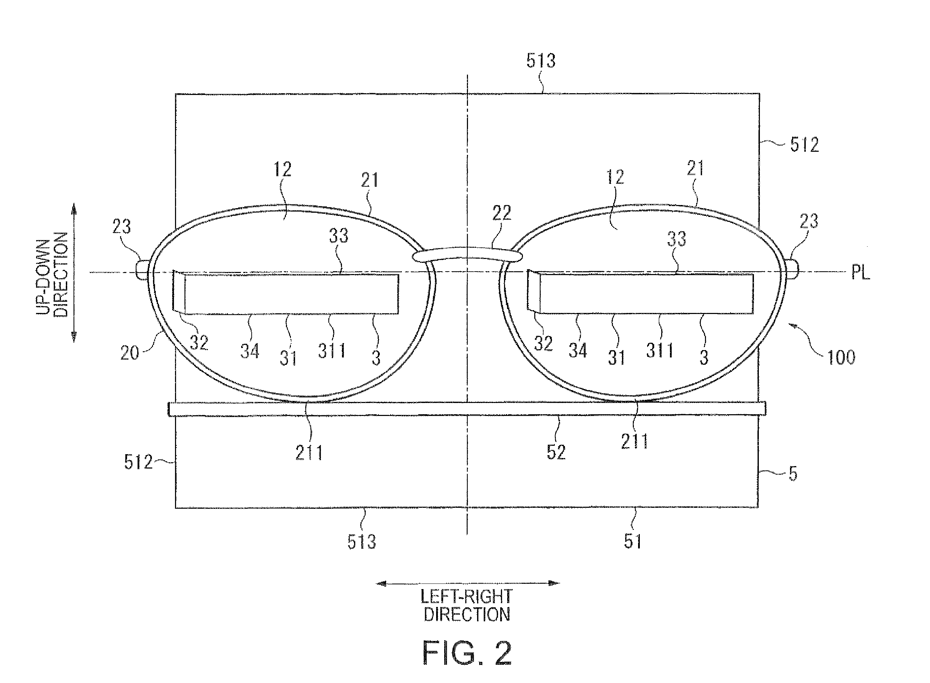

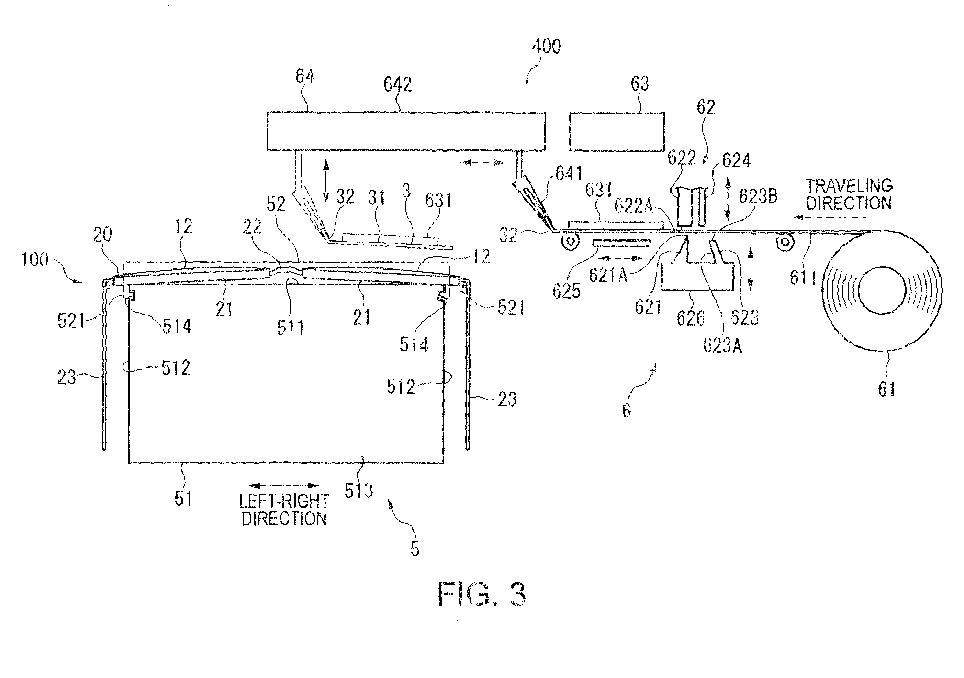

The spectacle lens 10 is produced by shaping and processing the progressive-power lens described above. The spectacle lenses 10 thus produced are attached to a spectacle frame 20 to become spectacles 100 (see Figs. 2 and 3).

The spectacle frame 20 has frame portions 21 each of which surrounds the corresponding attached spectacle lens 10 in the shape of frame, a bridge 22 for connecting the left and right frame portions 21, and temples 23 rotatably attached to the frame portions 21 via hinges.

For measurement of the downward eyeball movement, the spectacles 100 which contain examination spectacle lenses 12 made of acrylic resin and attached to the spectacle frame 20 are used. It is assumed in the following explanation that the examination spectacle lenses 12 have been attached to the spectacle frame 20.

### 2. Downward eyeball movement Measuring Jig

The measuring jig for measuring the length of the downward eyeball movement associated with the spectacle lens 10 is now explained. The downward eyeball movement herein refers to the distance between the distant eye point FP and the near eye point NP, i.e., the distance (length) L between the distant eye point line FL and the near eye point line NL in the up-down direction.

Tape-shaped masks 3 shown in Fig. 2 are used as the measuring jigs for measuring the length L of the downward eyeball movement. The measuring jigs are affixed to the examination spectacle lenses 12 made of acrylic resin and attached to the spectacle frame 20 to be purchased by the wearer. The tape-shaped masks 3 are formed by mending tapes available on the market (manufactured by Sumitomo 3M Limited), for example.

Each of the tape-shaped masks 3 includes a tape base 31 which has an adhesive portion 311 having one surface to be detachably affixed, and a folded portion 32 provided at one end of the tape base 31.

Examples of the width of the tape base 31 in the up-down direction under the condition affixed to the examination spectacle lens 12 involve 10 mm, 10.5 mm, 11 mm, 11.5 mm, 12 mm, 12.5 mm, 13 mm, 13.5 mm, 14 mm, 14.5 mm, and 15 mm. The width of the tape base 31 is defined by two opposed sides constituted by a first side 33 as one side and a second side 34 as the other side.

When the tape base 31 selected from various tape widths shown above for measurement has a width corresponding to the length of the downward eyeball movement appropriate for the wearer, the first side 33 corresponds to the distant eye point line FL of the examination spectacle lens 12. In this case, the second side 34 corresponds to the near eye point line NL of the examination spectacle lens 12.

The length of the tape base 31 in the left-right direction in the condition affixed to the examination spectacle lens 12 is not particularly limited as long as this length is smaller than the width of the examination spectacle lens 12 in the left-right direction.

For measurement, the tape base 31 having such a width as to allow alignment between the second side 34 and the near eye point line NL when the first side 33 of the tape base 31 is disposed aligned with the distant eye point line FL of the examination spectacle lens 12 is selected. The width of the tape base 31 corresponds to the length L of the downward eyeball movement.

The folded portion 32 is a non-adhesive portion formed by stacking folded parts of the adhesive portion 311. The folded portion 32 is held by a holding member 641 of a jig affixing device 400 described later when the tape-shaped mask 3 is affixed to or removed from the examination spectacle lens 12.

### 3. Structure of Jig Affixing Device

The jig affixing device 400 for affixing the tape-shaped mask 3 to the examination spectacle lens 12 is now explained.

As illustrated in Fig. 3, the jig affixing device 400 includes a spectacle hold unit 5 for holding the spectacles 100, a tape supply unit 6 for supplying the tape-shaped mask 3 to the spectacles 100 held by the spectacle hold unit 5, and a not-shown connect unit for connecting the spectacle hold unit 5 and the tape supply unit 6.

The spectacle hold unit 5 includes a base 51 as a parallelepiped component having a carrying surface 511 as an upper surface opposed to the examination spectacle lenses 12 when the spectacles 100 are placed on the spectacle hold unit 5, and a frame support member 52 freely movable in one direction along the carrying surface 511 of the base 51 and supporting the spectacles 100 at an appropriate position.

The base 51 has a pair of first side portions 512 opposed to each other and another pair of second side portions 513 opposed to each other, both pairs of which cross the carrying surface 511 at right angles. When the spectacles 100 are held, the frame portions 21 to which the examination spectacle lenses 12 are attached are placed on the carrying surface 511 in such a condition that the outside surfaces of the examination spectacle lenses 12 are positioned above in the vertical direction. In this case, the left and right temples 23 are disposed along the opposed first side portions 512. The carrying surface 511 is a flat surface, and the spectacles 100 placed on the carrying surface 511 are movable in the up-down direction of the spectacles 100 along the carrying surface 511. The carrying surface 511 has a positioning line PL for alignment with the distant eye point FP formed on the examination lens 12 as a mark.

Moreover, concave notches 514 are linearly formed on the upper regions of the first side portions 512, i.e., in the vicinity of the edges of the carrying surface 511 in a direction parallel with the carrying surface 511.

The frame support member 52 is a long component having at least a length larger than each length of the second side portions 513 in the left-right direction. Engaging portions 521 folded in U shape (hook shape) are provided at both ends of the frame support member 52. Each end of the engaging portions 521 engages with the corresponding notch 514 of the base 51 such that the engaging portions 521 are freely movable in the up-down direction of the examination spectacle lenses 12. The frame support member 52 moves while contacting lower sides 211 of the frame portions 21 to control the position of the spectacles 100. The frame support member 52 is controlled by a not-shown controller such that the distant eye point FP of the examination spectacle lens 12 can be automatically aligned with the positioning line PL on the carrying surface 511. The distant eye point FP of the examination spectacle lens 12 can be aligned with the positioning line PL on the carrying surface 511 by using a detection unit capable of detecting agreement between the distant eye point FP and the positioning line PL.

The tape supply unit 6 includes a feed section 61 for feeding a feed tape 611, and a cutter section 62 for cutting the feed tape 611 and folding the end of the cut feed tape 611 to form the folded portion 32, a bonding section 63 for bonding a contact bonding member 631 allowing contact bonding between the tape-shaped mask 3 formed by the cutter section 62 and the examination spectacle lens 12 to the tape-shaped mask 3, and a lens affixing section 64 for transferring the tape-shaped mask 3 to which the contact bonding member 631 has been bonded to the spectacles 100 and affixing the tape-shaped mask 3 to the examination spectacle lens 12.

The feed section 61 is a mending tape available on the market, and has a function of feeding the feed tape 611 to the cutter section 62.

The cutter section 62 includes a cutting portion 621 for cutting the feed tape 611, a cutter receiving portion 622 for receiving the cutting portion 621, a fold support portion 623 for forming the folded portion 32 at the end of the cut feed tape 611, a fold producing portion 624, and a fold finishing portion 625. The fold support portion 623, the fold producing portion 624, and the fold finishing portion 625 constitute a fold forming portion.

The cutting portion 621 projecting upward to a base 626 has a substantially triangular shape in the cross-sectional view with an acute angle positioned above, and the acute angle forms a cutter 621A. The cutting portion 621 is provided in such a position that the cutter can be located at a predetermined distance from the end of the feed tape 611, and is freely movable in the vertical direction with respect to the traveling direction of the feed tape 611. The predetermined distance is a distance corresponding to the length of the tape-shaped mask 3 (50 mm, for example).

The cutter receiving portion 622 has a contact surface 622A for receiving the cutter 621A of the cutting portion 621 and is fixed to a position opposed to the cutting portion 621 with the feed tape 611 sandwiched between the cutter receiving portion 622 and the cutting portion 621.

When the cutter 621A contacts the contact surface 622A of the cutter receiving portion 622 by the upward movement of the cutting portion 621 in the vertical direction (direction of approaching the cutter receiving portion 622), the cutter 621A cuts the feed tape 611. By this cutting process, the tape-shaped mask 3 having the tape base 31 and the folded portion 32 is produced.

The fold support portion 623 projecting upward to the base 626 in the vertical direction has a substantially trapezoidal shape in the cross-sectional view. The fold support portion 623 is disposed at a position shifted to the feed section 61 from the cutting portion 621 and formed integrally with the cutting portion 621. A wall surface 623A of the fold support portion 623 on the side opposed to the cutting portion 621 is inclined to the traveling direction of the feed tape 611 at an angle of at least 90 degrees. In this case, it is preferable that the angle formed by the wall surface 623A and an upper surface 623B of the fold support portion 623 is larger than 0 degree and equal to or smaller than 90 degrees. The distance between the fold support portion 623 and the cutting portion 621 corresponds to the half length of the folded portion 32 of the tape-shaped mask 3. For example, when the length of the folded portion 32 is 10 mm, the distance between the fold support portion 623 and the cutting portion 621 is 5 mm. The fold support portion 623 operates in a direction perpendicular to the traveling direction of the feed tape 611 (vertical direction) in cooperation with the cutting portion 621 and the base 626.

The fold producing portion 624 is a component having a flat plate shape and disposed at a position approximately above the fold support portion 623 in the vertical direction with the feed tape 611 sandwiched between the fold producing portion 624 and the fold support portion 623. The fold producing portion 624 is freely movable in the vertical direction with respect to the traveling direction of the feed tape 611. When the fold producing portion 624 and the fold support portion 623 move in directions of approaching each other, one of the surfaces of the fold producing portion 624 contacts the wall surface 623A of the fold support portion 623. After the cutting portion 621 cuts the feed tape 611, the fold producing portion 624 shifts downward in the vertical direction (direction of approaching the fold support portion 623) to contact the wall surface 623A with the end of the feed tape 611 sandwiched between the feed producing portion 624 and the wall surface 623A. As a result, the end of the feed tape 611 comes to follow the shapes of the wall surface 623A and the upper surface 623B, and thus a fold is formed at the end of the feed tape 611.

The fold finishing portion 625 is a component having a flat plate shape and disposed at a position shifted in the traveling direction of the feed tape 611 from the fold support portion 623 in such a condition that one of the surfaces of the fold finishing portion 625 is parallel with the feed tape 611. The fold finishing portion 625 is freely movable in parallel with the feed tape 611. After formation of the fold by the fold support portion 623 and the fold producing portion 624 at the end of the feed tape 611 and shift of the fold support portion 623 and the fold producing portion 624 in directions away from each other, the fold finishing portion 625 moves in the direction opposite to the traveling direction of the feed tape 611 having the fold, and presses the folded end such that the bonding portions of the feed tape 611 can be stacked (affixed). By this step, the folded portion 32 is produced.

The bonding section 63 bonds the contact bonding member 631 to the surface of the tape-shaped mask 3. The contact bonding member 631 is made of sponge, for example. It is preferable that the adhesive used herein has a bonding force sufficient for preventing separation between the contact bonding member 631 and the tape-shaped mask 3 when a force is not applied but easily allowing separation therebetween when a force is applied. The contact bonding member 631 may be attached to the surface of the tape-shaped mask 3 by using suction of air or the like or surface tension of liquid.

The lens affixing section 64 has the holding member 641 for holding the folded portion 32 of the tape-shaped mask 3, and a controller 642 for controlling the operation of the holding member 641.

The holding member 641 holds the folded portion 32, and shifts the folded portion 32 to a position above the spectacles 100 while holding the folded portion 32. Then, the holding member 641 descends toward the surface of one of the left and right examination spectacle lenses 12, and places the tape-shaped mask 3 on the selected examination spectacle lens 12 such that the distant eye point line FL of the examination spectacle lens 12 agrees with the first side 33 of the tape-shaped mask 3. The movement direction of the holding member 641 includes the left-right direction of the spectacles 100 for moving from the tape supply unit 6 to the spectacle hold unit 5, and the vertical direction for descending toward the examination spectacle lens 12.

The controller 642 controls the holding member 641 such that the holding member 641 can shift from the tape supply unit 6 to the examination spectacle lens 12 held by the spectacle hold unit 5 and perform the predetermined operations described above.

The not-shown connect unit connects the spectacle hold unit 5 and the tape supply unit 6 to fix the position of the spectacles 100 in the up-down direction. The spectacle hold unit 5 and the tape supply unit 6 are movable in the left-right direction of the spectacles 100 such that the positions of the left and right examination spectacle lenses 12 can be controlled when the tape-shaped masks 3 are affixed thereto.

Thus, the first side 33 of the tape-shaped mask 3 and the distant eye point line FL of the examination spectacle lens 12 can be aligned by moving the frame support member 52 and controlling the position of the spectacles 100 held by the spectacle hold unit 5.

### 4. Downward Eyeball Movement Measuring Method

Explained herein are a mask forming step for forming the tape-shaped mask 3 by using the jig affixing device 400, a mask affixing step for affixing the tape-shaped mask3 to the examination spectacle lens 12, and a measuring step for measuring downward eyeball movement by using the affixed tape-shaped mask 3.

Initially, as illustrated in Fig. 3, the spectacle frame 20 of the spectacles 100 is opened, and the spectacles 100 are placed such that the examination spectacle lenses 12 can be opposed to the carrying surface 511 of the base 51, that is, the temples 23 can be disposed along the first side portions 512. Each of the examination spectacle lenses 12 has the distant eye pint FP determined beforehand with a mark provided at the corresponding position. Thus, when the spectacles 100 are placed on the base 51, the frame support member 52 is shifted by the not-shown controller such that the distant eye point FP and the positioning line PL can be automatically aligned. As a result, the positioning line PL aligned with the distant eye point FP becomes the distant eye point line FL of the examination spectacle lens 12.

### 4-1. Mask Forming Step

Then, the tape-shaped mask 3 is formed. Initially, a mending tape whose width has the same length as the expected length L of the downward eyeball movement of the wearer is attached to the feed section 61 of the tape supply unit 6. Then, the feed tape 611 is supplied from the feed section 61, and the feed tape 611 is cut at an appropriate position to form the initial folded portion 32. The cutting process herein is performed to form the folded portion 32 at the end of the feed tape 611 for the initial use of the feed tape 611, and the same cutting process is repeated at the intervals of the predetermined distance from the end of the feed tape 611 in the following steps.

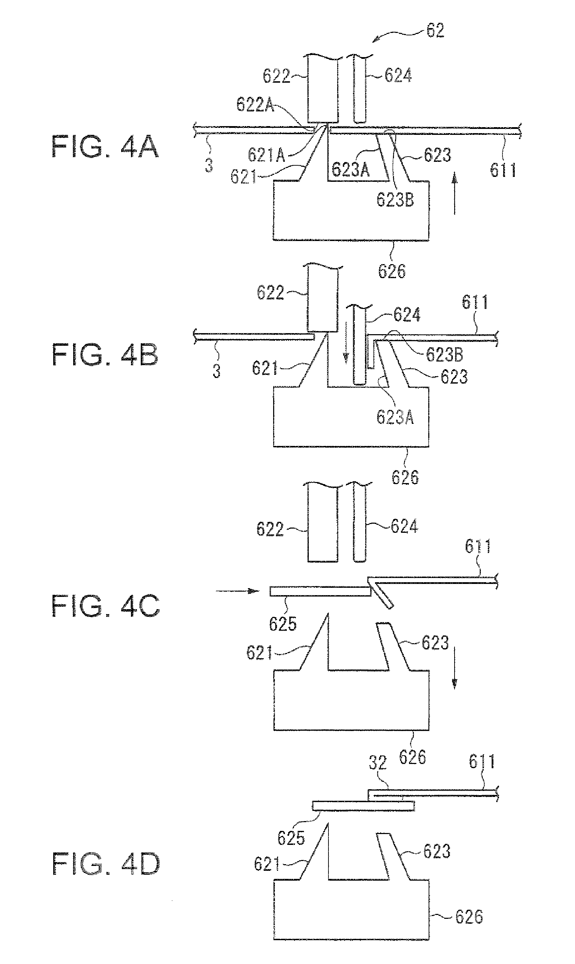

The cutting process is now discussed in more detail with reference to Figs. 4A through 4D.

Fig. 4A illustrates a condition in which the feed tape 611 is cut by the cutter 621A of the cutting portion 621 shifted upward in the vertical direction and brought into contact with the contact surface 622A of the cutter receiving portion 622. Immediately after the feed tape 611 is cut, the fold producing portion 624 moves downward in the vertical direction to form a fold at the end of the feed tape 611 along the wall surface 623A and the upper surface 623B of the fold support portion 623 as illustrated in Fig. 4B. Then, the fold support portion 623 and the fold producing portion 624 are shifted in directions away from each other, and the fold finishing portion 625 is moved in the direction opposite to the traveling direction of the feed tape 611 to push a fold end formed by the fold of the feed tape 611 as illustrated in Fig. 4C. As a result, the folded portion 32 shown in Fig. 4D is produced.

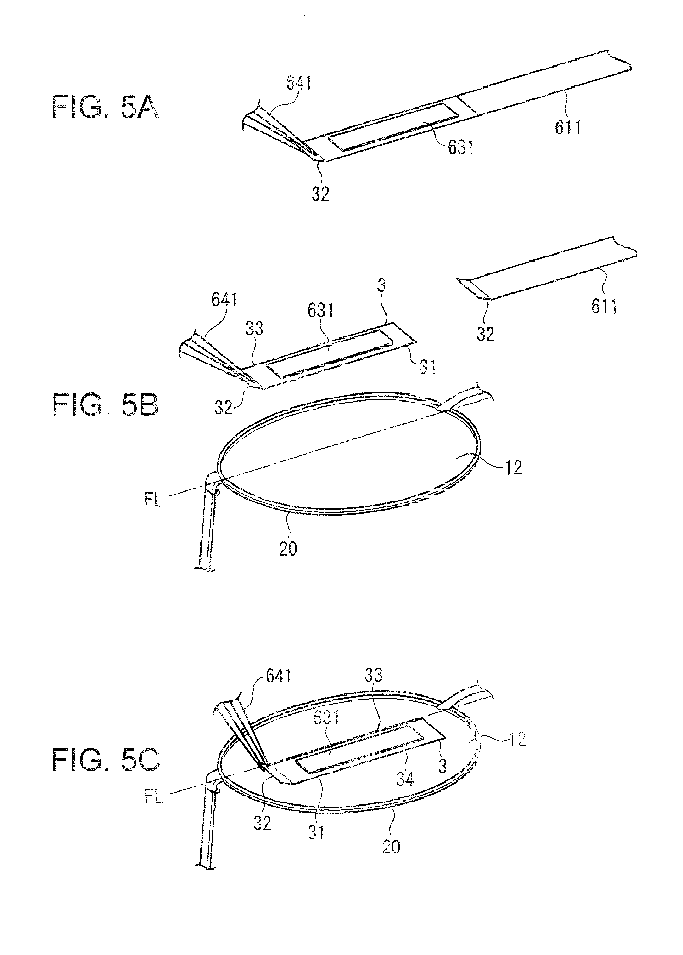

The feed tape 611 having the folded portion 32 after the cutting process is transferred to a position where the folded portion 32 can be held by the holding member 641. Then, the holding member 641 holds the folded portion 32, and the bonding section 63 places the contact bonding member 631 to which the adhesive has been applied on the surface of the feed tape 611 as illustrated in Fig. 5A. By this step, the contact bonding member 631 is bonded to the feed tape 611. Then, the cutting process is again performed to produce the tape-shaped mask 3 (see Fig 5B).

### 4-2. Mask Affixing Step

Next, the tape-shaped mask 3 is shifted to a position above the examination spectacle lens 12 by the holding member 641 as illustrated in Fig. 5B.

Then, the holding member 641 is controlled to move downward in the vertical direction (direction of approaching the examination spectacle lens 12), and the tape-shaped mask 3 is placed on the examination spectacle lens 12 such that the distant eye point line FL can be aligned with the first side 33 of the tape-shaped mask 3 (see Fig. 5C).

The tape-shaped mask 3 is contact-bonded to the surface of the examination spectacle lens 12 by the contact bonding member 631. After the tape-shaped mask 3 is affixed to the examination spectacle lens 12, the contact bonding member 631 is removed.

After the tape-shaped mask 3 is affixed to one of the examination spectacle lenses 12 by this method, another tape-shaped mask 3 is similarly affixed to the other examination spectacle lens 12 by adjusting the position of the base 51.

### 4-3. Measuring Step (Determining Step)

The wearer wears the spectacles 100 which have the tape-shaped masks 3 affixed to the examination spectacle lenses 12, and checks the vision positions for long-distance view and short-distance view.

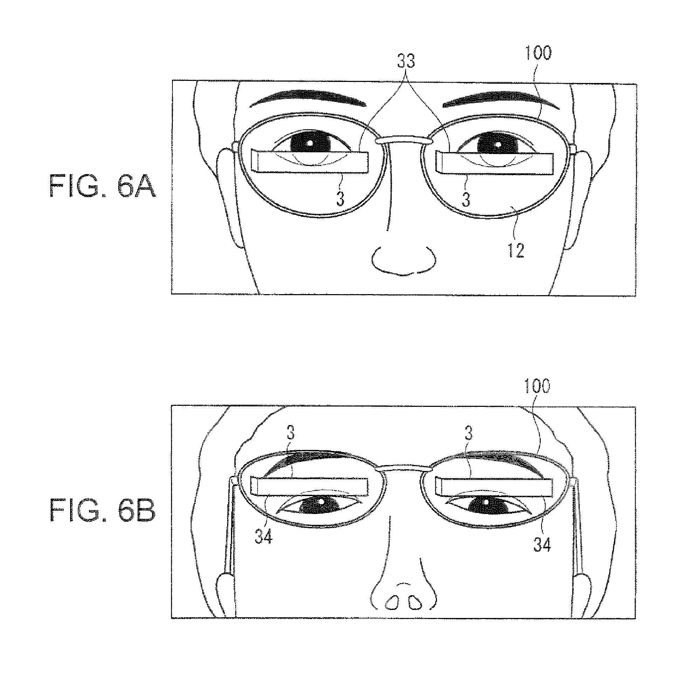

More specifically, the wearer wearing the spectacles 100 checks whether the pupil center (vision) in the condition of front view passes the first side 33 of the tape-shaped mask 3 (checking distant eye point) as illustrated in Fig. 6A. In this embodiment, the distant eye point has been determined as the position of the pupil center in the condition of front view beforehand, and it is thus obvious that the vision passes the first side 33. When the vision does not pass the first side 33, the distant eye point line FL is re-established before repeating the measurement.

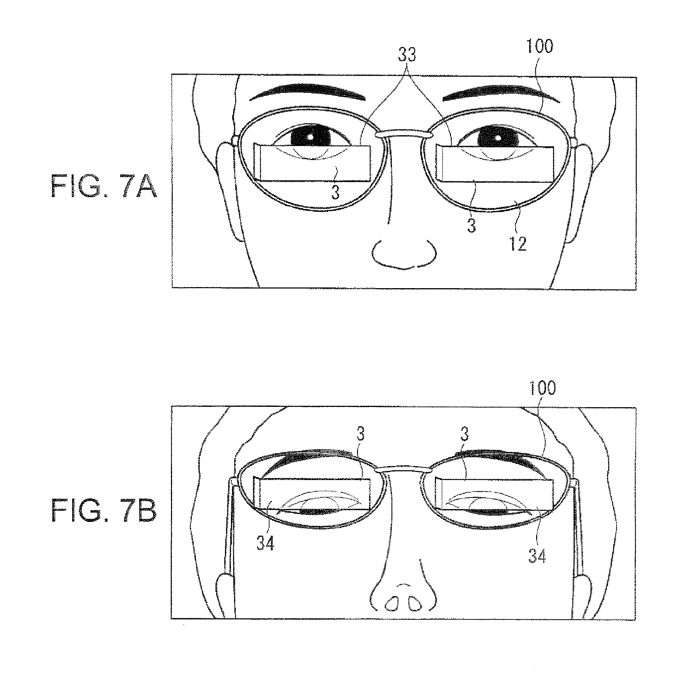

Next, the wearer checks whether the pupil center (vision) for reading (short-distance view) passes the second side 34 of the tape-shaped mask 3 (checking near eye point position) as illustrated in Fig. 6B. Figs. 7A and 7B show an example of the tape-shaped mask 3 having a large width (see Fig. 7A). As illustrated in Fig. 7B, the vision for the short-distance view is blocked by the tape-shaped mask 3 and cannot be recognized. In this case, it is determined that the near eye point NP does not agree with the second side 34 of the tape-shaped mask 3. Since the disagreement is caused by the too large width of the tape-shaped mask 3, the measurement is repeated using the tape-shaped mask having a smaller width. When the vision is located at a position away from the second side 34 of the tape-shaped mask 3, the tape-shaped mask 3 is too small. In this case, the measurement is repeated using the tape-shaped mask 3 having a larger width. By this method, whether the near eye point NP agrees with the second side 34 of the tape-shaped mask 3 is determined to select the tape-shaped mask 3 having the optimum width. The width of the tape-shaped mask 3 finally selected corresponds to the length L of the downward eyeball movement of the wearer.

### 5. Advantages of First Embodiment

According to the first embodiment, the following advantages can be offered.

In this embodiment, the length L of the downward eyeball movement is measured for each wearer by using the tape-shaped mask 3 having the required width. The person practicing the measurement is only required to affix the tape-shaped mask 3 having various widths to the surface of the examination spectacle lens 12, and thus can perform the measurement easily and at low cost. Particularly, the measurement can be easily conducted at a shop or the like, which is highly advantageous.

During the measurement, the wearer wears the spectacle frame to be purchased and worn by the wearer. Thus, not only the movements of the head and the eyes of the wearer (viewing action) but also the position of the wearer can be taken into account for the measurement. Accordingly, the optimum length L of the downward eyeball movement for each wearer can be highly accurately measured while considering the spectacle frame and the behavior (position) of the wearer for viewing an object.

The wearer is only required to wear the spectacles 100 to which the tape-shaped mask 3 has been affixed and determine whether the vision passes the first side 33 and the second side 34 of the tape-shaped mask 3 for long-distance view and short-distance view. Thus, the measurement can be more easily performed. The person practicing the measurement may determine whether the vision of the wearer passes these sides by visual inspection. In this case, the accuracy of the measurement rises with the increased number of determination.

When the vision does not pass the first side 33 and the second side 34 of the tape-shapedmask 3, it is only required to remove the tape-shaped mask 3 and affix another tape-shape mask 3 having a different width. Thus, the person practicing the measurement can easily perform the measurement without special skills.

The tape-shaped mask 3 has the folded portion 32 formed by the adhesive portions affixed to each other. Thus, the holding member 641 can easily hold the folded portion 32, and executes the operations of shifting, affixing, and removing the tape-shaped mask 3 easily and efficiently. Moreover, the adhesive portion 311 of the tape-shaped mask 3 has only an adhesive force allowing free attachment and detachment. Thus, the tape-shaped mask 3 can be easily removed from the examination spectacle lens 12.

The jig affixing device 400 can cut the feed tape 611 and form the folded portion 32 by using the tape supply unit 6. Thus, the tape-shaped mask 3 can be easily and efficiently produced, and the steps and the required time can be reduced.

The spectacle hold unit 5 and the tape supply unit 6 are positioned beforehand with respect to the up-down direction of the spectacles 100. Thus, the first side 33 of the tape-shaped mask 3 and the distant eye point line FL can be aligned only by shifting the frame support member 52. Accordingly, positioning can be easily carried out for each spectacle frame.

For affixing the tape-shaped mask 3, the contact bonding member 631 is bonded to the surface of the tape-shaped mask 3 on the side opposite to the adhesive portion 311 of the tape base 31. By this method, wrinkles and warps of the tape-shaped mask 3 can be prevented when the tape-shaped mask 3 is placed on the examination spectacle lens 12. Moreover, the tape-shaped mask 3 can be securely affixed to the examination spectacle lens 12 by the weight of the contact bonding member 631.

### Second Embodiment

A second embodiment of the invention is now described with reference to the drawings. The spectacle lens used in the second embodiment (progressive-power lens) has the same structure as that of the first embodiment, and the same explanation is not repeated.

### 1. Downward Eyeball Movement Measuring Jig

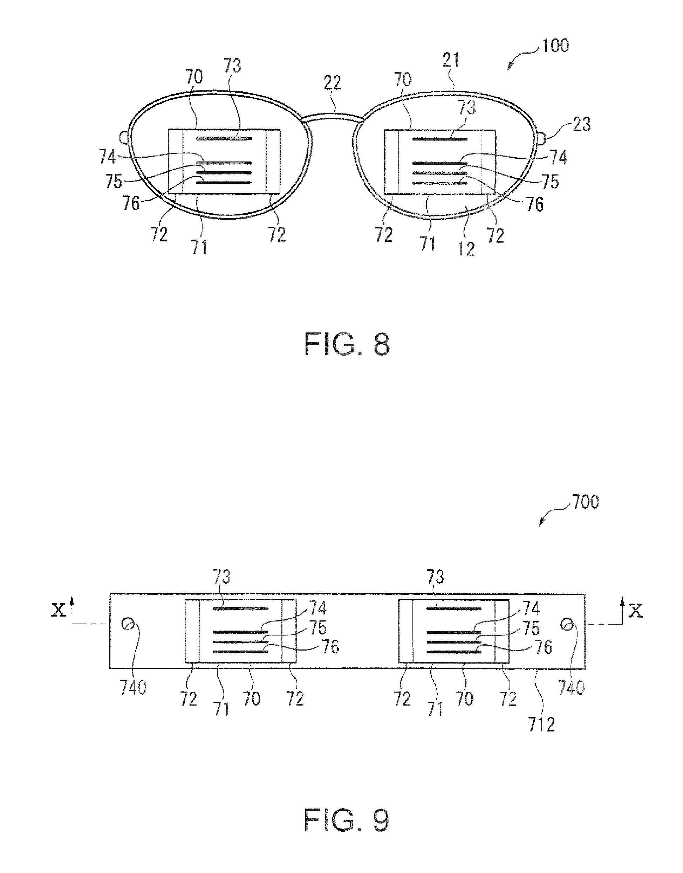

Fig. 8 illustrates downward eyeball movement measuring jigs affixed to the examination spectacle lenses 12 according to the second embodiment. In the second embodiment, tape-shaped masks 70 shown in Fig. 8 are used as the measuring jigs for measuring the length of the downward eyeball movement.

Each of the tape-shaped masks 70 is made of see-through type transparent film, and includes a rectangular adhesive portion 71 whose one surface is detachably affixed, and a pair of non-adhesive portions 72 provided adjacent to two opposed sides of the adhesive portion 71. The see-through type transparent film has transparency sufficient for reading characters through the tape-shaped mask 70.

The adhesive portion 71 is a colorless and transparent component having a plurality of marks on the surface. These marks are constituted by an FP line 73 indicating the distant eye point FP, a first NP line 74, a second NP line 75, and a third NP line 76 provided in an area expected to contain the near eye point. The length of the adhesive portion 71 in the left-right direction is not particularly limited as long as it is smaller than the width of the examination spectacle lens 12 in the left-right direction.

The FP line 73 is a straight line which corresponds to the distant eye point line FL passing the distant eye point FP of the examination spectacle lens 12 and linearly extending in the left-right direction of the examination spectacle lens 12. The vision of the wearer passes the distant eye point FP when the wearer wearing the spectacles 100 has horizontal view. The distant eye point FP is determined beforehand.

The first NP line 74, the second NP line 75, and the third NP line 76 are straight lines provided in the area expected to contain the near eye point NP, and extending in parallel with the FP line 73. Each of the widths of these lines 74 through 76 is 1 mm, for example, but not specifically limited. Each space between the lines 74 through 76 is 2 mm, for example, but not specifically limited. The first NP line 74, the second NP line 75, and the third NP line 76 have different opaque colors: red for the first NP line 74, green for the second NP line 75, and blue for the third NP line 76.

The tape shaped mask 70 is affixed in such a position that the FP line 73 coincides with the distant eye point line FL passing the distant eye point FP of the examination spectacle lens 12. The near eye point NP is set at the position of the visible closest line for the wearer in the condition of short-distance view. In this case, the length of the downward eyeball movement corresponds to the distance between the FP line 73 and the position of the selected line.

The non-adhesive portion 72 is an area not bonded to the examination spectacle lens 12. The tape-shaped mask 70 can be easily removed from the examination spectacle lens 12 by holding the non-adhesive portion 72.

### 2. Structure of Jig Affixing Device

A jig affixing device for affixing the tape-shaped mask 70 to the examination spectacle lens 12 is now explained.

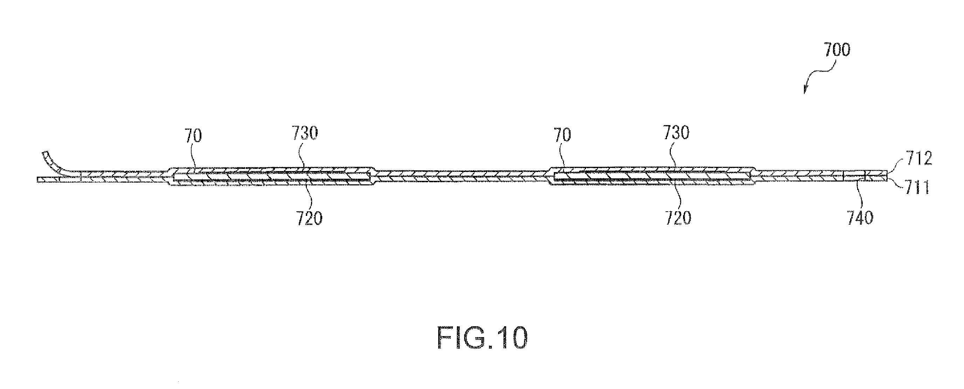

Initially, a measurement tape placed on the jig affixing device is discussed. As illustrated in Figs. 9 and 10, a measurement tape 700 includes a base film 711 and a cover film 712 laminated on the based film 711, and each of the tape-shaped masks 70 is sandwiched between the base film 711 and the cover film 712. The base film 711 is bonded to the tape-shaped mask 70 by a first adhesive layer 720. A second adhesive layer 730 is provided on the cover film side surface of the tape-shaped mask 70. The second adhesive layer 730 is formed in the area corresponding to the adhesive portion 71 of the tape-shaped mask 70. Thus, the area not having the second adhesive layer 730 corresponds to the non-adhesive portion 72 of the tape-shaped mask 70. The second adhesive layer 730 may be formed on the entire area of one of the surfaces of the tape-shaped mask 70. In this case, the non-adhesive portion 72 can be produced by providing printing for lowering the adhesion of the adhesive on the area corresponding to the non-adhesive portion 72.

The steps for removing the tape-shaped mask 70 from the measurement tape 700 and affixing the tape-shaped mask 70 to the examination spectacle lens 12 are now specifically described.

Initially, the cover film 712 is separated from the base film 711. The adhesion strength between the second adhesive layer 730 and the cover film 712 is lower than the adhesion strength between the tape-shaped mask 70 and the second adhesive layer 730. Thus, the cover film 712 can be separated from the second adhesive layer 730. As a result, the second adhesive layer 730 is exposed on the surface of the tape-shaped mask 70.

Then, the examination spectacle lens 12 made of acrylic resin is pressed against the second adhesive layer 730 exposed on the surface of the tape-shaped mask 70 to affix the tape-shaped mask 70 to the examination spectacle lens 12.

Subsequently, the base film 711 is removed from the tape-shaped mask 70. The adhesive strength between the tape-shaped mask 70 and the first adhesive layer 720 is lower than the adhesive strength between the base film 711 and the first adhesive layer 720. Thus, the tape-shaped mask 70 is separated from the first adhesive layer 720. As a result, the tape-shaped mask 70 is affixed to the examination spectacle lens 12 via the second adhesive layer 730.

After the downward eyeball movement is measured by using the tape-shaped mask 70 affixed to the examination spectacle lens 12, the tape-shaped mask 70 is removed from the examination spectacle lens 12. In this case, the adhesive strength between the examination spectacle lens 12 and the second adhesive layer 730 is lower than the adhesive strength between the tape-shaped mask 70 and the second adhesive layer 730. Thus, the examination spectacle lens 12 is separated from the second adhesive layer 730.

By this method, all of the tape-shaped mask 70 and the second adhesive layer 730 are removed, and the examination spectacle lens 12 is returned to the original state.

The films and the adhesives used herein have the following structure. The balance of the adhesive strength can be adjusted by applying release agent to the respective films.

For example, it is preferable that the base film 711 is a sheet containing at least one type of material selected from a group of polyethylene terephthalate, polyolefin, polystyrene, polyvinyl chloride and others. It is also preferable that the thickness of the base film 711 lies in the range from 3 µm to 500 µm.

It is preferable that the cover film 712 is a sheet containing at least one type of material selected from a group of polyethylene terephthalate, polyolefin, polystyrene, polyvinyl chloride and others, with release agent applied to the sheet. It is also preferable that the thickness of the cover film 712 lies in the range from 3 µm to 500 µm.

It is preferable that the tape-shaped mask 70 is a sheet containing at least one type of material selected from a group of polyethylene terephthalate, polyolefin, polystyrene, polyvinyl chloride and others, with release agent applied to the sheet. It is also preferable that the thickness of the tape-shaped film 70 lies in the range from 3 µm to 500 µm.

It is preferable that the adhesive used as the first adhesive layer 720 is a layer containing at least one type of material selected from a group of acrylic resin, rubber, urethane resin, silicon resin, vinyl ether, and epoxy resin. It is also preferable that the thickness of the first adhesive layer 720 lies in the range from 1 µm to 200 µm.

It is preferable that the adhesive used as the second adhesive layer 730 is a layer containing at least one type of material selected from a group of acrylic resin, rubber, urethane resin, silicon resin, vinyl ether, and epoxy resin. It is also preferable that the thickness of the second adhesive layer 730 lies in the range from 1 µm to 200 µm.

The measurement tape 700 has through holes 740 penetrating the base film 711 and the cover film 712 in the vicinity of both end edges. The through holes 740 engage with engaging portions 840 of a jig affixing device 800 described later to fix the measurement tape 700 when the measurement tape 700 is placed on the jig affixing device 800.

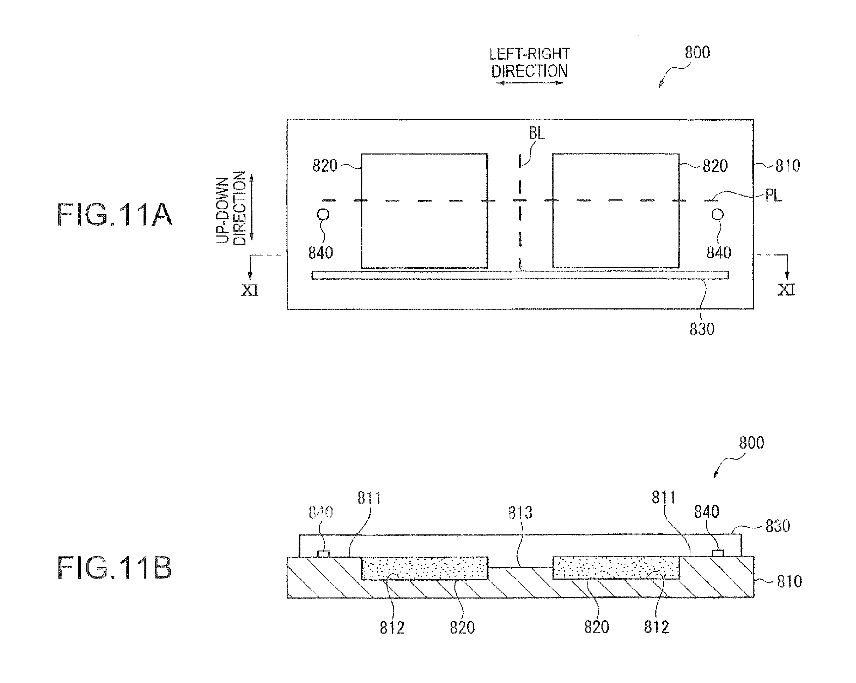

The structure of the jig affixing device 800 is now explained. As illustrated in Fig. 11A, the jig affixing device 800 includes a spectacle hold stand 810 holding the spectacles 100, lens carrying portions 820 provided on the upper surface of the spectacle hold stand 810, a frame support member 830 for controlling the position of the spectacles 100, and the engaging portions 840 engaging with the through holes 740 of the measurement tape 700.

As illustrated in Fig. 11B, the spectacle hold stand 810 is a rectangular parallelepiped component having a carrying surface 811 as an upper surface opposed to the examination spectacle lenses 12 when the spectacles 100 are placed on the jig affixing device 800. The carrying surface 811 has a pair of rectangular concaves 812 for receiving the lens carrying portions 820 at the positions opposed to the examination spectacle lenses 12, and an intermediate portion 813 sandwiched between the pair of concaves 812 and having an upper surface positioned lower than the height of the carrying surface 811.

The lens carrying portions 820 are rectangular areas made of low impact resilience material and received by the pair of the concaves 812. It is preferable that the low impact resilience material has resiliency sufficient for preventing damage to the surfaces of the examination spectacle lenses 12, such as urethane.

The positioning line PL extending in the left-right direction for alignment with the FP line 73 of the tape-shaped mask 70 and a frame reference line BL extending in the up-down direction for alignment with the center line of the frame 20 on the intermediate portion 813 are provided on the surfaces of the spectacle hold stand 810 and the lens carrying portions 820.

The frame support member 830 is a long component parallel with the positioning line PL, and is movable in the up-down direction of the examination spectacle lens 12. The frame support member 830 moves while contacting the lower sides 211 of the frame portions 21 to control the position of the spectacles 100. The frame support member 830 is manually adjustable.

The engaging portions 840 are convexed at positions outside the pair of the lens carrying portions 820. The engaging portions 840 engage with the through holes 740 of the measurement tape 700 to fix the measurement tape 700.

Thus, design is determined such that the positional relationship between the positioning line PL and the engaging portions 840 of the jig affixing device 800 agrees with the positional relationship between the FP line 73 and the through holes 740 of the measurement tape 700.

### 3. Downward Eyeball Movement Measuring Method

Explained herein are a mask affixing step for affixing the tape-shaped mask 70 to the examination spectacle lens 12 by using the jig affixing device 800, and a measuring step for measuring the downward eyeball movement by using the affixed tape-shaped mask 70.

### 3-1. Mask Affixing Step

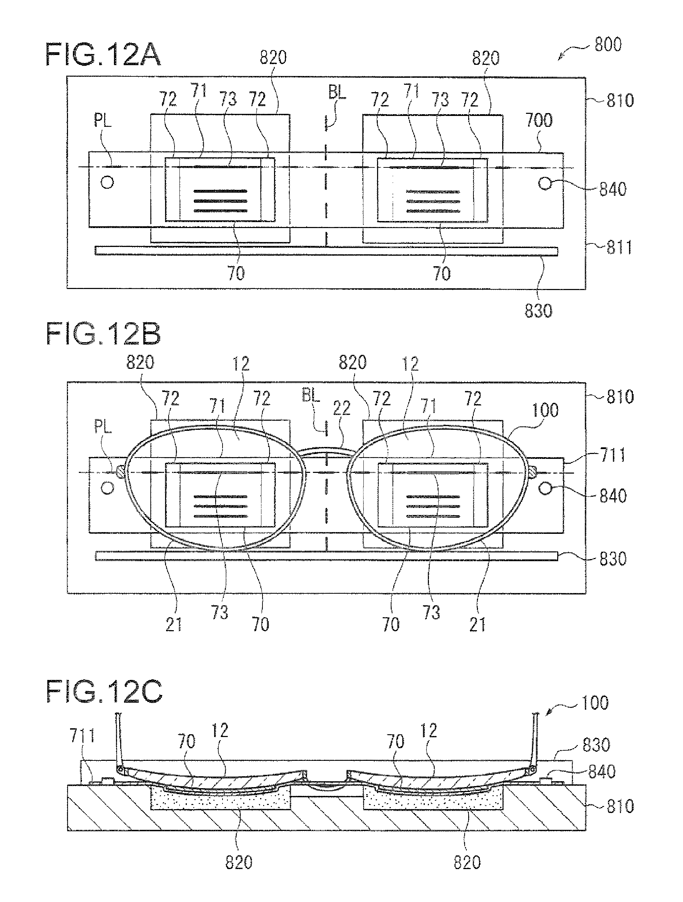

As illustrated in Fig. 12A, the measurement tape 700 is placed on the carrying surface 811 of the jig affixing device 800. In this step, the pair of the through holes 740 of the measurement tape 700 are fitted to the engaging portions 840 of the jig affixing device 800 to fix the position of the measurement tape 700. In addition, alignment between the positioning line PL and the FP line 73 is checked.

Then, the frame support member 830 is positioned. The spectacle frame 20 of the spectacles 100 is opened, and the spectacles 100 are placed on the carrying surface 811 of the jig affixing device 800 in such a direction that the outer surfaces of the examination spectacle lenses 12 can contact the measurement tape 700 (see Fig. 12B). In this case, the spectacles 100 are positioned such that the center of the frame 20 lies on the frame reference line BL. The examination spectacle lens 12 has the distant eye point FP determined beforehand, and a mark is provided at the corresponding position. Thus, fine adjustment of the position of the spectacles 100 is performed such that the distant eye point FP of the examination spectacle lens 12 can be aligned with the positioning line PL or the FP line 73 with the frame support member 830 contacting the frame portions 21.

After the position of the frame support member 830 is determined, the spectacles 100 are temporarily removed from the jig affixing device 800. Then, the cover film 712 of the measurement tape 700 is separated from the base film 711. As a result, the second adhesive layer 730 of the tape-shaped mask 70 is exposed.

Subsequently, each outer surface of the pair of the examination spectacle lenses 12 is pressed against the corresponding low impact resilience material of the pair of the lens carrying portions 820 such that the spectacles 100 contact the frame reference line BL and the frame support member 830. As a result, the examination spectacle lens 12 and the tape-shaped mask 70 are bonded to each other via the second adhesive layer 730 as illustrated in Fig. 12C.

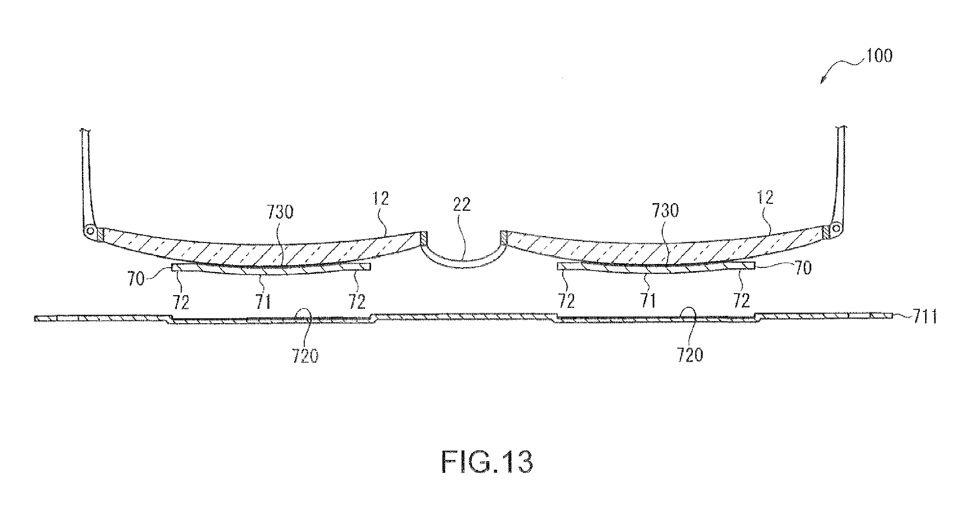

Then, the spectacles 100 are separated from the base film 711 as illustrated in Fig. 13. In this step, the spectacles 100 are released from the base film 711 by separation between the tape-shaped mask 70 and the first adhesive layer 720, and thus the tape-shaped mask 70 remains affixed to the examination spectacle lens 12 via the second adhesive layer 730. As a result, each of the tape-shaped mask 70 is affixed to the spectacles 100 in the condition shown in Fig. 8, and the condition allows measurement of the downward eyeball movement.

### 3-2. Measuring Step

Next, the measuring step for measuring the downward eyeball movement by using the tape-shaped mask 70 affixed to the examination spectacle lens 12 is explained.

The wearer wears the spectacles 100 having the tape-shaped masks 70 affixed to the examination spectacle lenses 12, and checks the positions of the visions for long-distance view and the short-distance view.

More specifically, the wearer wearing the spectacles 100 checks whether the pupil center (vision) of the wearer viewing the front passes the FP line 73 of the tape-shaped mask 70. Then, the wearer wearing the spectacles 100 checks which position receives the pupil center (vision) of the wearer in reading (viewing a near object). In this embodiment, the first NP line 74, the second NP line 75, and the third NP line 76 are provided. Thus, the wearer determines the line closest to the position receiving the vision of the wearer.

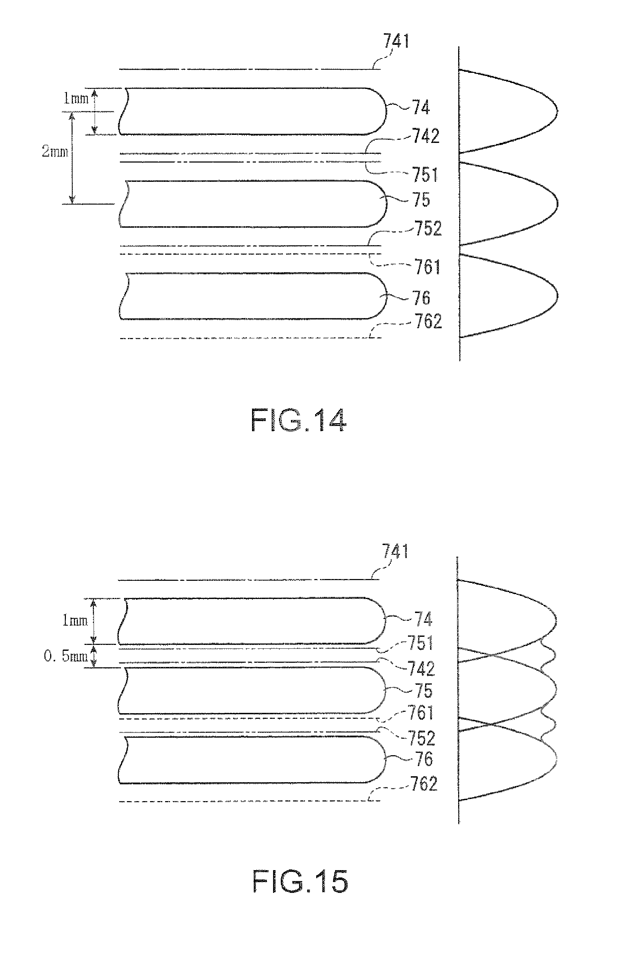

The lines actually viewed by the wearer wearing the spectacles 100 are herein explained. For the wearer wearing the spectacles 100 in the condition of short-distance view, the first NP line 74, the second NP line 75, and the third NP line 76 are viewed as wider and more blurred lines than the actual lines (reflection colors). Fig. 14 illustrates the first NP line 74, the second NP line 75, and the third NP line 76, and schematic absorbancy spectrum of the respective lines and their peripheries. The absorbancy spectrum becomes the maximum at the centers of the first NP line 74, the second NP line 75, and the third NP line 76, and decreases as the directions are away from the respective lines. That is, the spectrum exhibits such gradation which becomes the darkest at the respective centers of the first NP line 74, the second NP line 75, and the third NP line 76, and becomes lighter toward the peripheries. The gradation of the first NP line 74 ends at a first boundary line 741 and a second boundary line 742. The gradation of the second NP line 75 ends at a third boundary line 751 and a fourth boundary line 752. The gradation of the third NP line 76 ends at a fifth boundary line 761 and a sixth boundary line 762. In this case, a new white line (transmission light) is visible between the second boundary line 742 and the third boundary line 751. Similarly, a new white line (transmission light) is visible between the fourth boundary line 752 and the fifth boundary line 761. Thus, more lines than the three lines of the first NP line 74, the second NP line 75, and the third NP line 76 actually provided can be recognized (a combination of reflection lights and transmission lights).

Accordingly, the wearer viewing five lines constituted by the first NP line 74, the second NP line 75, and the third NP line 76 actually provided on the tape-shaped mask 70, and the new white lines other than the three lines 74 through 76 (the combination of reflection lights and transmission lights) determines the line closest to the position receiving the vision of the wearer in the five lines for the measurement. The distance between the selected line and the FP line 73 corresponds to the length of the downward eyeball movement of the wearer.

### 4. Advantages of Second Embodiment

According to the second embodiment, the following advantages can be provided.

In this embodiment, the length of the downward eyeball movement is measured by using the tape-shaped mask 70 which has the FP line 73 indicating the distant eye point, and the first NP line 74, the second NP line 75, and the third NP line 76 indicating the near eye point.

According to this structure, the wearer wears the spectacles 100 to be purchased and worn by the wearer during the measurement. Thus, not only the movements of the head and the eyes of the wearer (viewing action) but also the position of the wearer can be checked during the measurement. Accordingly, the optimum length of the downward eyeball movement for each wearer can be highly accurately measured while considering the spectacle frame and the behavior of the wearer for viewing an object.

The wearer is only required to wear the spectacles 100 to which the tape-shaped masks 70 have been affixed and determine the line closest to the vision of the short-distance view in the plural lines provided on the examination spectacle lenses 12. Thus, the measurement can be easily conducted without imposing burden on the wearer.

Particularly, in the second embodiment, the wearer can see additional lines other than the three lines of the first NP line 74, the second NP line 75, and the third NP line 76 provided on the tape-shaped mask 70 during the actual measurement. Generally, the distance between the lines decreases and thus the accuracy of the measurement improves as the number of the lines increases. Accordingly, highly accurate measurement can be performed by using a small number of lines.

In addition, this method is highly advantageous since the number of lines visible for the wearer can be controlled by adjusting the widths and distances between the first NP line 74, the second NP line 75, and the third NP line 76.

The jig affixing device 800 in the second embodiment has the lens carrying portions 820 made of low impact resilience material. Thus, it is only required to push the examination spectacle lens 12 against the tape-shaped mask 70 fixed to the carrying surface 811. Accordingly, the tape-shaped mask 70 can be easily affixed.

Particularly, this method is highly advantageous since the person practicing the measurement at the shop or the like can affix the tape-shaped mask 70 only by pushing the examination spectacle lens 12 against the tape-shaped mask 70 as easy preparation for the measurement.

In the second embodiment, the intermediate portion 813 having a height lower than the heights of the upper surfaces of the carrying surface 811 and the lens carrying portions 820 is provided between the pair of the lens carrying portions 820. According to this structure, the bridge 22 is fitted to the intermediate portion 813 when the spectacles 100 are placed on the carrying surface 811. Thus, the position of the spectacles 100 can be stabilized.

The tape-shaped mask 70 has the non-adhesive portion 72 to be held when the tape-shaped mask 70 is separated from the examination spectacle lens 12. Thus, removal of the tape-shaped mask 70 is facilitated.

### Modified Example

While the embodiments of the invention have been illustrated and described, various modifications and improvements may be made without departing from the scope of the invention. Therefore, it is intended that the invention is not limited to the particular embodiments described herein, but that the invention will include all embodiments falling within the scope of the appended claims.

For example, while the frame support member 52 is automatically shifted in the first embodiment, the frame support member 52 may be manually positioned. In this case, the controller for controlling the frame support member 52 is not needed, and thus the structure can be simplified.

According to the first embodiment, the base 51 is shifted in the left-right direction after the tape-shaped mask 3 is affixed to one of the examination spectacle lenses 12. Then, another tape-shaped mask 3 is affixed to the other examination spectacle lens 12. However, the controller 642 of the lens affixing section 64 may be extended such that the holding member 641 can reach both of the left and right examination spectacle lenses 12, for example. In this case, the necessity for moving the base 51 is eliminated, and only adjustment of the position of the holding member 641 is required. Thus, positioning can be further stabilized.

According to the first embodiment, the folded portion 32 is formed by folding the end of the tape-shaped mask 3. However, the folded portion may be any part as long as it can be held by the holding member 641. For example, a part having no adhesion may be affixed to the adhesive portion 311 within a predetermined distance from the end of the tape base 31. In this case, it is only required to affix the part to the adhesive portion 311 of the tape base 31. Thus, the structure of the tape supply unit can be simplified.

According to the first embodiment, the carrying surface 511 of the base 51 is a flat surface. However, a convex portion engaging with the bridge 22 may be formed on the carrying surface 511 so as to support the spectacle frame 20 (spectacles 100) in a more stable manner. In this case, the spectacle frame 20 is freely movable along the carrying surface 511 and the convex portion. According to this structure, movement of the spectacles 100 in the left-right direction is prevented, and the position of the spectacles 100 is more stabilized. Thus, the tape-shaped mask 3 can be more accurately affixed to the examination spectacle lens 12.

According to the second embodiment, each space between the first NP line 74, the second NP line 75, and the third NP line 76 formed on the tape-shaped mask 70 is set at 2 mm. However, the width of the space is not limited to this length. When the space is 0.5 mm, for example, the wearer can see a new line (reflection light) having a combination of the colors between the respective lines. More specifically, the wearer sees a new yellow line between the first NP line 74 (red) and the second NP line 75 (green), and a new cyan line between the second NP line 75 (green) and the third NP line 76 (blue). These new lines are produced by the blurring portion of the first NP line 74 and the blurring portion of the second NP line 75 having a narrow space therebetween and thus overlapping with each other as illustrated in Fig. 15.

According to this structure, lines having different colors can be seen only by adjusting the widths, spaces, and colors of the respective lines. Since various types of colors are used, determination for the measurement can be easily made by the wearer without imposing heavy burden on the wearer.

According to the second embodiment, the first NP line 74, the second NP line 75, and the third NP line 76 provided on the tape-shaped mask 70 have different colors. However, these colors may be the same color. In this case, the types of ink can be decreased, and thus cost reduction can be achieved.

According to the second embodiment, design is determined such that the positional relationship between the positioning line PL and the engaging portions 840 of the jig affixing device 800 agrees with the positional relationship between the FP line 73 and the through holes 740 of the measurement tape 700. However, the engaging portions 840 of the jig affixing device 800 may be so constructed as to be movable in the up-down direction. In this case, designs and manufactures of the jig affixing device 800 and the measurement tape 700 are freely determined, and thus flexibility increases.

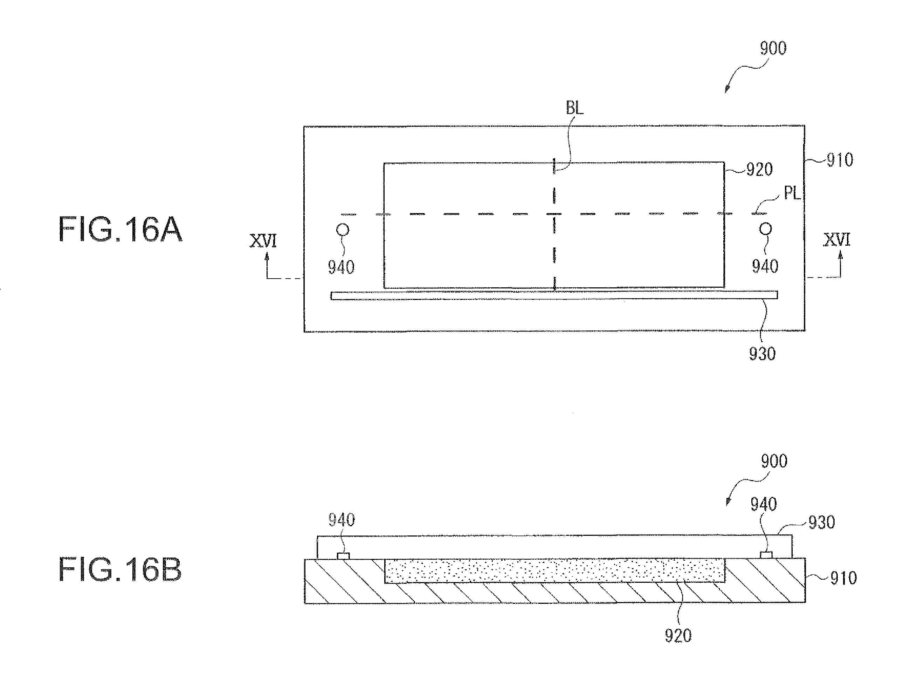

According to the second embodiment, a pair of the lens carrying portions 820 are provided on the jig affixing device 800, and the intermediate portion 813 is interposed between the pair of the lens carrying portions 820. However, a spectacle carrying portion 920 in the shape connecting the pair of the lens carrying portions 820 may be provided as illustrated in Figs. 16A and 16B. The spectacle carrying portion 920 is made of low impact resilience material. Thus, when the spectacles 100 are placed on the spectacle carrying portion 920, the bridge 22 of the spectacles 100 is pressed against the low impact resilience material. As a result, the spectacles 100 can be positioned with high stability.

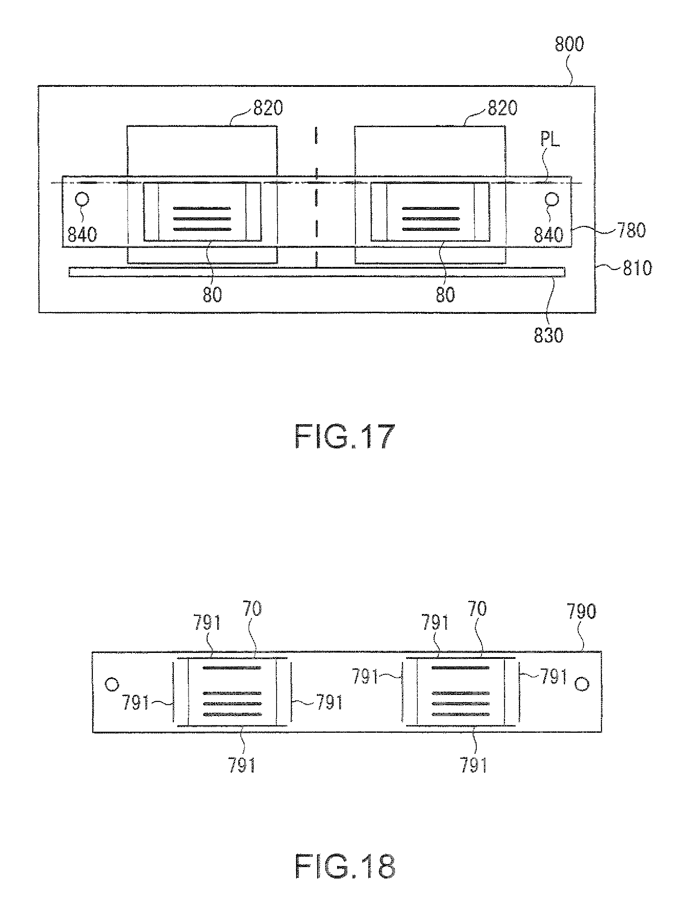

According to the second embodiment, the FP line 73 indicating the distant eye point line FL is provided on the tape-shaped mask 70. However, one side of the tale-shaped mask may be used as the distant eye point line as illustrated in Fig. 17. More specifically, one side of a tape-shaped mask 80 is aligned with the positioning line PL of the jig affixing device 800. In this case, the line indicating the distant eye point need not be provided on the tape-shaped mask 80. Thus, the manufacture of the tape-shaped mask 80 can be simplified.

According to the second embodiment, the measurement tape 700 has the tape-shaped mask 70 between the base film 711 and the cover film 712. However, the measurement tape 700 is not required to have this structure. For example, notches 791 may be provided on one base film 790 such that the tape-shaped mask 70 can be cut from the base film 790 as illustrated in Fig. 18. In this case, it is preferable that the long notches 791 are formed to reduce the connection portion so as to easily cut away the tape-shaped mask 70. For example, only the four corners of the tape-shaped mask 70 are connected to the base film 790. According to this structure, the material cost can be reduced.

According to the embodiments described herein, the downward eyeball movement is measured by affixing the tape-shaped mask to the examination spectacle lens 12. However, the tape-shaped mask may be affixed to the spectacles 100 which have progressive-power lenses (spectacle lenses 10) designed based on the measured downward eyeball movement and attached to the spectacle frame 20. In this case, the downward eyeball movement of the designed progressive-power lenses can be checked, and the accuracy can be increased. Accordingly, the spectacle lenses suited for the viewing action of each wearer can be produced.

The downward eyeball movement measuring jig according to the invention which easily measures downward eyeball movement associated with a progressive-power lens can be used in various places such as shops dealing in spectacles.

## Claims

1. A downward eyeball movement measuring jig which measures downward eyeball movement corresponding to a distance between a distant eye point (FP) and a near eye point (NP) of a spectacle lens (12) attached to a spectacle frame (21) to be worn by a wearer of the spectacle lens, comprising:
a mask (3; 70; 80) affixable on the surface of an examination spectacle lens (12) attached to the spectacle frame and having a reference line passing the distant eye point (FP) and extending in the horizontal direction.

2. The downward eyeball movement measuring jig according to claim 1, wherein:
the mask (3; 70; 80) has a width corresponding to the expected length of the downward eyeball movement of the wearer and two opposed sides; and
the reference line corresponds to one (33) of the two opposed sides (33, 34) of the mask.

3. The downward eyeball movement measuring jig according to claim 1, wherein:
the mask (3; 70; 80) has two opposed sides (33, 34); and
the reference line is disposed at a position away from the two opposed sides.

4. The downward eyeball movement measuring jig according to claim 2, wherein:
the other side (34) of the two opposed sides (33, 34) of the mask (3; 70; 80) is to be disposed directed to the near eye point (NP); and
the mask is a tape-shaped mask which has a tape base (31), and an adhesive portion (311; 71) disposed on the side of the tape base to face the examination spectacle lens (12) and detachably affixable to the examination spectacle lens.

5. The downward eyeball movement measuring jig according to claim 2, wherein:
the mask (70; 80) has a plurality of lines (73, 74, 75, 76) to be provided on the surface of the examination spectacle lens in an area expected to contain the near eye point (NP) in such directions parallel with the reference line; and
the mask (70; 80) is a tape-shaped mask which has a tape base, and an adhesive portion disposed on the side of the tape base to face the examination spectacle lens (12) and detachably affixable to the examination spectacle lens.

6. The downward eyeball movement measuring jig according to claim 5, wherein the plural lines (73, 74, 75, 76) have colors different from one another.

7. A jig affixing device (400) which affixes the downward eyeball movement measuring jig according to claim 4 to an examination spectacle lens (12), comprising:
a spectacle hold unit (5) which holds spectacles (100) having the examination spectacle lens (12) attached to a spectacle frame (21); and
a tape supply unit (6) which supplies the tape-shaped mask (3; 70; 80) to the spectacles held by the spectacle hold unit,
wherein
the spectacle hold unit (5) has a base (51) opposed to the examination spectacle lens (12), and a frame support member (52) attached to the base in such a condition as to be movable in a direction perpendicular to a horizontal vision area passing an eye point of the examination spectacle lens and extending in the horizontal direction to support the spectacle frame (21), and
the tape supply unit (6) has a cutter member (62) which cuts the tape-shaped mask (3, 70; 80), and a lens affixing member (641) which transfers the tape-shaped mask cut by the cutter member to the spectacles (100) held by the spectacle hold unit (5) and affixes the tape-shaped mask such that one of the sides (33, 34) of the tape-shaped mask can be aligned with the horizontal vision area.

8. The downward eyeball movement measuring jig according to claim 4, wherein an end of the tape-shaped mask (3; 70; 80) is formed by folding the adhesive portion (311; 71) such that the folded parts (32; 72) of the adhesive portion can be stacked with each other.

9. The jig affixing device according to claim 7, wherein the cutter member (62) has a fold forming portion (624) which folds the end of the tape-shaped mask (3; 70).

10. A method for measuring downward eyeball movement corresponding to a distance between a distant eye point (FP) and a near eye point (NP) of a spectacle lens (12) attached to a spectacle frame (21) to be worn by a wearer of the spectacle lens, comprising:
preparing a tape-shaped mask (3; 70; 80) which has a width corresponding to the expected length of the downward eyeball movement of the wearer for the surface of an examination spectacle lens (12) attached to the spectacle frame (21), positioning the tape-shaped mask such that one (33) of two opposed sides of the tape-shaped mask passes the distant eye point (FP) of the examination spectacle lens (12), and affixing the tape-shaped mask (3, 70; 80) to the examination spectacle lens with the other side (34) of the tape-shaped mask located close to the near eye point (NP) of the examination spectacle lens (12); and
determining whether the near eye point (NP) of the examination spectacle lens (12) agrees with the other side (34) of the tape-shaped mask, and the determination is made by the wearer wearing spectacles (100) which has the examination spectacle lens (12) attached to the spectacle frame (21) with the tape-shaped mask (3; 70; 80) affixed to the examination spectacle lens,
wherein
when it is determined that the near eye point does not agree with the other side of the tape-shaped mask (3; 70; 80), determination is repeated after removing the tape-shaped mask from the examination spectacle lens and affixing another tape-shaped mask (3; 70; 80) having a different width.
